Europäisches Patentamt

European Patent Office

Office européen des brevets

(19)

(11) Publication number: **0 233 493**

**A2**

(12) **EUROPEAN PATENT APPLICATION**

(21) Application number: **87100742.3**

(22) Date of filing: **20.01.87**

(51) Int. Cl.⁴: **C12N 11/04** , **C12P 19/38** , **C12P 19/40** , **C12P 19/28**

(30) Priority: **21.01.86 JP 10811/86**
**17.01.87 JP 8732/86**

(43) Date of publication of application:
**26.08.87 Bulletin 87/35**

(84) Designated Contracting States:
**BE CH DE ES FR GB IT LI NL SE**

(71) Applicant: **Yamasa Shoyu Kabushiki Kaisha**
**10-1, Araoi-Cho 2-Chome**
**Choshi-Shi Chiba-Ken(JP)**
Applicant: **KANSAI PAINT CO. LTD.**
**33-1, Kanzaki-cho**
**Amagasaki-shi Hyogo-ken(JP)**
Applicant: **JGC Corporation**
**2-1, Ohte-machi 2-chome**
**Chiyoda-Ku Tokyo-To(JP)**

(72) Inventor: **Ueno, Junji**
**29-10, Karako-Cho**
**Choshi-Shi Chiba-Ken(JP)**
Inventor: **Fujishima, Tetsuro**
**8539-8, Kobatake-Shin-Machi**
**Choshi-Shi Chiba-Ken(JP)**
Inventor: **Iida, Takamitsu**
**3269-30, Okazaki**
**Hiratsuka-Shi Kanagawa-Ken(JP)**
Inventor: **Sakamoto, Masahiro**
**15-8, Koma 2-Chome Oiso-Cho**
**Naka-Gun Kanagawa-Ken(JP)**

(74) Representative: **Dr. Elisabeth Jung Dr. Jürgen**
**Schirdewahn Dipl.-Ing. Claus Gernhardt**
**P.O. Box 40 14 68 Clemensstrasse 30**
**D-8000 München 40(DE)**

(54) **Process for producing ribonucleosides.**

(57) The present invention provides a process for producing ribonucleosides by reacting a base donor with a ribose compound in the presence of nucleoside phosphorylase to form N-glycosidic linkage between the base moiety of the base donor and the ribose moiety of the ribose compound, which process is characterized by the use of an immobilized nucleoside phosphorylase preparation obtained by subjecting a mixture of an enzyme source having nucleosidase activity in addition to nucleoside phosphorylase activity and a photo-curable resin having a photosensitive radical at any position of the prepolymer chain structure thereof to irradiation with actinic rays, whereby the activity of the nucleosidase concomitantly present in the enzyme source is inhibited.

# PROCESS FOR PRODUCING RIBONUCLEOSIDES

BACKGROUND OF THE INVENTION

The present invention relates to a process for producing ribonucleosides. More particularly, the invention relates to a process for producing ribonucleosides by the use of a nucleoside phosphorylase preparation having nucleosidase activity in addition to nucleoside phosphorylase activity whereby the base moiety and the ribose moiety are bonded through N-glycosidic linkage, in which process ribonucleosides are obtained stably and efficiently over a long period of time by immobilizing beforehand an enzyme source having nucleosidase activity in addition to nucleoside phosphorylase activity with a photo-curable resin inclusively thereby to inhibit the activity of the nucleosidase concomitantly present in the enzyme source.

Known processes for producing ribonucleosides by enzymatically reacting a base donor and a ribose compound are, for example, processes for producing a variety of purine-ribonucleosides (Japanese Patent Pub. Nos. 24475/1968, 28954/1968, 28956/1968, IIII6/1970, I4957/1973, etc.) and processes for producing ribavirin or derivatives thereof (Japanese Patent Pub. No. 36960/1983, Japanese Patent Laid-Open Pub. Nos. I46593/1982, I90396/1983, 2I6696/1983, etc.).

All of these processes are considered to be based on the use of enzymatically active substances comprising microorganisms per seprimarily having nucleoside phosphorylase activity or substances obtained therefrom. In these processes, however, the problems of concomitance of nucleosidase in the enzymatically active substance and the resulting reduction in yield of the desired ribonucleosides are not recognized, nor are solutions therefor taken into consideration.

For a method of removing or inhibiting unnecessary enzyme activities in the substance containing a plurality of enzymes, on the other hand, a method of mutating microorganisms used for the production of the enzymes to inhibit production of unnecessary enzymes, a method of adding an inhibitor against unnecessary enzymes to the reaction system during enzyme reaction and the like have so far been proposed.

It is also known in the art to subject enzymes or microbial cells immobilized with a photo-curable resin to various enzyme reactions (for example, Japanese Patent Pub. Nos. 40/1980, 20676/1980, 4I9I8/1982, 3673/1983, 3674/1983, 3I96/1984, 32II7/1984, I367I/1985, and Japanese Patent Laid-Open Pub. No. II8797/1982).

The last publication discloses a process for producing adenine arabinosides by immobilizing microbial cells capable of producing adenine arabinoside from uracil arabinoside and adenine with a photo-curable resin and contacting the immobilized cells with a solution of pyrimidine arabinoside and an adenine donor such as adenine dissolved in an aqueous solvent containing 5% by weight or more dimethyl sulfoxide.

The characteristic feature of this prior invention is deemed to reside in the addition of a specific quantity of an organic solvent to the reaction solution thereby to improve the solubility of the starting compounds and the end product and also in the immobilization of the microbial cells with a photo-curable resin thereby to prevent reduction in enzyme activity of the microbial cells in the presence of the organic solvent added and under the high temperature conditions which are considered to be optimum reaction conditions (the reaction temperature employed in Examples being 60°C).

The advantages of this prior invention seem to be attained only by the combination of the addition of a specific organic solvent to the reaction solution and the immobilization of the microbial cells used with a photo-curable resin.

While it is not clear whether or not the microbial cells used have nucleosidase activity or, if they have nucleosidase activity, whether or not the activity adversely affects the yield of adenine arabinoside, arabinonucleosides are generally considered to have higher resistance to nucleosidase than ribonucleosides.

Further, while enzymes are generally deemed to act in different ways in the presence and absence of an organic solvent, this prior invention discloses the enzyme reaction of photo-curable resin immobilized microorganisms in the presence of an organic solvent.

In the production of ribonucleosides by the use of a nucleoside phosphorylase preparation whereby the base moiety and the ribose moiety are bonded through N-glycosidic linkage, a serious problem encountered when nucleosidase is present in the preparation is that the substrates and/or end product are/is decomposed, resulting in reduced production rate of the desired ribonucleosides.

It would not be easy, however, to apply conventional methods of removing or inhibiting enzyme activity in order to solve such a problem.

2

For instance, the method wherein an organism as an enzyme source is caused to undergo mutation to reduce only the nucleosidase activity thereof is difficult since this method not only involves a cumbersome procedure but also accompanies reduction in nucleoside phosphorylase activity in some cases. When a ribonucleotide is used as a ribose compound, in particular, the preparation must contain, in addition to nucleoside phosphorylase, phosphatase or nucleotidase so that it would be more difficult to remove only nucleosidase activity from such preparation by the method described above.

Further, the method utilizing an inhibitor is inapplicable to the production of ribonucleosides since an inhibitor that specifically inhibits nucleosidase activity without affecting nucleoside phosphorylase activity has not yet been found as far as we are aware.

In light of the foregoing, the problem to be solved by the present invention is to improve, in the production of ribonucleosides by the use of an enzyme source containing a plurality of enzymes such as nucleoside phosphorylase and nucleosidase, for example, microbial cells, the yield of ribonucleosides by specifically inhibiting only nucleosidase activity without lowering nucleoside phosphorylase activity in the reaction system.


## SUMMARY OF THE INVENTION

As a result of our intensive researches on methods of specifically inhibiting unnecessary nucleosidase activity in the production of ribonucleosides by the use of a nucleoside phosphorylase preparation having nucleosidase activity in addition to nucleoside phosphorylase activity whereby the base moiety and the ribose moiety are bonded through N-glycosidic linkage, we have found that nucleosidase activity is specifically inhibited in an immobilized nucleoside phosphorylase preparation comprising a photo-crosslinked polymer prepared by admixing an enzyme source having nucleosidase activity in addition to nucleoside phosphorylase activity with a photo-curable resin and irradiating the mixture with actinic rays. On the basis of this finding, we have arrived at the present invention.

Thus, the present invention provides a process for producing ribonucleosides by reacting a base donor with a ribose compound in the presence of nucleoside phosphorylase to form N-glycosidic linkage between the base moiety of the base donor and the ribose moiety of the ribose compound, which process is characterized by the use of an immobilized nucleoside phosphorylase preparation obtained by subjecting a mixture of an enzyme source having nucleosidase activity in addition to nucleoside phosphorylase activity and a photo-curable resin having a photosensitive radical at any position of the prepolymer chain structure thereof to irradiation with actinic rays.

The present invention as described above will further lead to the following advantages.

Admittedly, nucleosidase activity concomitant with nucleoside phosphorylase activity in the preparation can be inhibited, for example, by conducting a reaction at around 55 to 70°C based on the difference in heat stability between the two enzymes. In this method, however, nucleoside phosphorylase undergoes such rapid heat inactivation that the method cannot be regarded as beneficial from the point of view of commercial operability.

In accordance with the present invention, it has become possible to carry out the enzymatic reaction even at relatively low reaction temperatures while inhibiting nucleosidase activity simply by immobilizing an enzyme source with a photo-curable resin.

The present invention has further made possible a continuous use of an immobilized nucleoside phosphorylase preparation over a long period of time in the production of ribonucleosides by increasing the heat stability of nucleoside phosphorylase.

The reduction in nucleoside phosphorylase activity during immobilization which tends to accompany conventional immobilization processes is not observed in the process of the present invention. The inhibition of nucleosidase activity makes it possible to produce ribonucleosides in a high yield.

In view of the overall advantages set forth hereinabove, the process of this invention is one of the best processes for producing ribonucleosides by the action of nucleoside phosphorylase.


## DETAILED DESCRIPTION OF THE INVENTION

The "ribonucleosides" to be produced by the process of the present invention are compounds wherein an aromatic heterocyclic base and ribofuranosyl group are bonded through N-glycosidic linkage and refer generically to compounds that can be produced by the enzymatic reaction of nucleoside phosphorylase from the corresponding base donor and ribose donor.

Specific examples of such ribonucleosides are 9-β-D-ribofuranosyl purines, l-β-D-ribofuranosyl pyrimidines, l-β-D-ribofuranosyl-l,2,4-triazoles, l-β-D-ribofuranosyl imidazoles, deazapurine β-D-ribofuranosides, azapurine β-D-ribofuranosides, azapyrimidine β-D-ribofuranosides, and l-β-D-ribofuranosyl pyridines.

More specifically, examples of the 9-β-D-ribofuranosyl purines are those having a substituent or substituents (e.g., amino, substituted amino, hydroxyl, oxo, mercapto, acyl, alkyl, substituted alkyl, alkoxyl and a halogen) at one or two of the l-, 2-, 6-and 8-positions of the purine base, such as adenosine, guanosine, inosine, xanthosine, 6-mercaptopurine β-D-ribofuranoside, 6-thioguanosine, $N^6$-alkyl-or acyladenosine, 2-alkoxyadenosine, 2-thioadenosine and 2,6-diaminopurine β-D-ribofuranoside.

Examples of the l-β-D-ribofuranosyl pyrimidines are those having the same substituent as are enumerated above at one or more of the 2-, 4-and 5-positions of the pyrimidine base, such as cytidine, uridine, ribothymidine, 5-halogenouridine (e.g., 5-fluorouridine, 5-iodouridine, 5-chlorouridine and 5-bromouridine), 5-halogenocytidine (e.g., 5-fluorocytidine and 5-chlorocytidine), 5-trihalogenomethyluridine (e.g., 5-trifluoromethyluridine), 4-thiocytidine, 4-thiouridine, $N^4$-acylcytidine and 5-halogenovinyluridine (e.g., 5-bromovinyluridine).

Examples of the l-β-D-ribofuranosyl-l,2,4-triazoles include those having a substituent at the 3-position of l,2,4-triazole base, such as l-β-D-ribofuranosyl-l,2,4-triazole-3-carboxamide, l-β-D-ribofuranosyl-l,2,4-triazole-3-carboxylic acid, l-β-D-ribofuranosyl-l,2,4-triazole-3-alkyl carboxylate.

Examples of the l-β-D-ribofuranosyl imidazoles are those having substituents at the 4-and 5-positions of the imidazole base, such as 5-amino-l-ribofuranosyl imidazole-4-carboxamide and 4-carbamoyl-l-ribofuranosyl imidazolium-5-oleate.

Examples of the deazapurine β-D-ribofuranosides include l-deazaadenosine, 3-deazaadenosine, 3-deazaguanosine, 7-deazaadenosine and 7-deazaguanosine, or deazapurine β-D-ribofuranosides having the same substituents as have been enumerated for the 9-β-D-ribofuranosyl purines while examples of the azapurine β-D-ribofuranosides include 8-azaadenosine.

Examples of the azapyrimidine β-D-ribofuranosides are 5-azaribothymidine, 5-azacytidine and 6-azauridine while examples of l-β-D-ribofuranosyl pyridines are 3-deazauridine, l-β-D-ribofuranosyl nicotinic acid and l-β-D-ribofuranosyl nicotinic acid amide.

The term "nucleoside phosphorylase activity" as used herein refers to an enzyme activity whereby N-glycosidic linkage can be formed between the base moiety of the base donor and the ribose moiety of the ribose compound. Namely, in the present invention, the enzyme activity which catalyzes the above reaction is called nucleoside phosphorylase activity, and this term is intended to include not only nucleoside phosphorylase activity in its proper sense but also the activity of transferases such as nucleoside-N-glycosyl transferase.

The term "nucleosidase activity" herein refers to the enzyme activity which hydrolyzes the N-glycosidic linkage in the ribonucleoside to produce a ribose and a base.

In view of the foregoing, the term "enzyme source" may further contain some other enzymes such as nucleotidase, phosphatase and deaminase.

Enzyme sources of any origin, for example, those derived from tissues of animals and plants or microbial cells (including spores), can be used as the nucleoside phosphorylase preparation of the present invention, those derived from microorganisms being preferred from the standpoint of commercial production efficiency. Nucleoside phosphorylase is present in a wide variety of microorganisms.

Some examples of the genera of the microorganisms used as enzyme sources in the prior art processes for producing ribonucleosides in the presence of nucleoside phosphorylase are as follows:

(l) the genus Brevibacterium
(2) the genus Corynebacterium
(3) the genus Arthrobacter
(4) the genus Micrococcus
(5) the genus Bacillus
(6) the genus Flavobacterium
(7) the genus Microbacterium
(8) the genus Xanthomonas
(9) the genus Pseudomonas
(l0) the genus Achromobacter
(ll) the genus Escherichia
(l2) the genus Staphylococcus
(l3) the genus Serratia
(l4) the genus Bacterium

4

(15) the genus <u>Proteus</u>
(16) the genus <u>Cellulomonas</u>
(17) the genus <u>Enterobacter</u>
(18) the genus <u>Mycoplana</u>
(19) the genus <u>Vibrio</u>
(20) the genus <u>Erwinia</u>
(21) the genus <u>Klebsiella</u>
(22) the genus <u>Aeromonas</u>
(23) the genus <u>Alteromonas</u>
(24) the genus <u>Kurthia</u>
(25) the genus <u>Protaminobacter</u>
(26) the genus <u>Salmonella</u>
(27) the genus <u>Citrobacter</u>
(28) the genus <u>Sporosarcina</u>
(29) the genus <u>Alkaligenes</u>
(30) the genus <u>Actinoplanes</u>
(31) the genus <u>Micromonospora</u>
(32) the genus <u>Nocardia</u>
(33) the genus <u>Streptoverticillium</u>
(34) the genus <u>Streptosporangium</u>
(35) the genus <u>Thermoactinomyces</u>
(36) the genus <u>Streptomyces</u>
(37) the genus <u>Saccharomyces</u>
(38) the genus <u>Mycotorula</u>
(39) the genus <u>Candida</u>

More specifically, some examples of the species of the microorganisms which fall under these genera and the strains of such species are as follows:

(1-1)
<u>Brevibacterium</u> <u>acetylicum</u> AT-6-7 FERM P-6305/ATCC 3931I/KAIST 830927-I08I0

(1-2)
<u>Brevibacteriumacetylicum</u>  ATCC 953

(1-3)
<u>Brevibacterium</u> <u>acetylicum</u>  ATCC 954

(1-4)
<u>Brevibacterium</u> <u>acetylicum</u>  ATCC 2I665

(1-5)
<u>Brevibacterium</u> <u>imperiale</u>  ATCC 8365

(1-6)
<u>Brevibacterium</u> <u>ammoniagenes</u>  ATCC 687I/KFCC II740

(2-1)
<u>Corynebacterium</u> <u>equi</u>  IAM I038/KCTC I298

(2-2)
<u>Corynebacterium</u> <u>michiganense</u>  ATCC 7429

(3-1)
<u>Arthrobacter</u> <u>globiformis</u>  IFO I2I37/ATCC 80I0

(3-2)
<u>Arthrobacter</u> <u>citreus</u>  IFO I2957/ATCC II624/KCTC I00I

(3-3)
<u>Arthrobacter</u> <u>simplex</u>  IFO I2069/ATCC 6946/KFCC 35400

(4-1)
<u>Micrococcus</u> <u>luteus</u>  ATCC 4698/IAM I056/KCTC I056

(4-2)
<u>Micrococcus</u> <u>luteus</u>  IAM I030

(4-3)
<u>Micrococcus</u> <u>luteus</u>  ATCC 398

(4-4)
<u>Micrococcus</u> <u>varians</u>  IFO 3765/ATCC 399

5

(4-5)

Micrococcus roseus  IFO 3768/ATCC l86

(5-l)

Bacillus subtilis  ATCC l4593/KFCC 3542l/KCTC l023

(5-2)

Bacillus cereus  IAM l029/ATCC 2l634

(5-3)

Bacillus  sphaericus  IFO 334l

(5-4)

Bacillus brevis  ATCC 8l85/KFCC ll7ll

(6-l)

Flavobacterium arborescens  IFO 3750/ATCC 4358

(6-2)

Flavobacterium lutesens  IFO 3084/ATCC 253ll

(6-3)

Flavobacterium lutesens  IFO 3085

(6-4)

Flavobacterium esteroaromaticum  IFO 375l/ATCC 809l

(6-5)

Flavobacterium rhenanum  CCM 298

(7-l)

Microbacterium  thermosphactum  IFO l2l67

(=Brochothrix  thermosphacta  ATCC ll509)

(8-l)

Xanthomonas oryzae  IFO 33l2

(=Xanthomonas campestris  ATCC 2l754)

(8-2)

Xanthomonas campestris  ATCC 738l

(9-l)

Pseudomonas desmolytica  J-4-2  FERM P-6307/ATCC 393l0

(9-2)

Pseudomonas schuylkilliensis  IAM l05l

(9-3)

Pseudomonas ovalis  IAM l002

(9-4)

Pseudomonas dacunhae  IAM l089

(9-5)

Pseudomonas diminuta  ATCC ll568

(l0-l)

Achromobacter eurydice  BE-3-3  FERM P-6304/ATCC 393l2

(l0-2)

Achromobacter parvulus  IFO l3l82/NRRL B-2395

(l0-3)

Achromobacter xerosis  IFO l2668

(l0-4)

Achromobacter lactium  CCM 69

(ll-l)

Escherichia coli  IFO 330l/KFCC 32396

(ll-2)

Escherichia coli  IAM l268/ATCC ll303

(ll-3)

Escherichia coli  IFO l3500/ATCC 9637/KCTC l039

(ll-4)

Escherichia coli  IFO l3540/ATCC l28l4

(ll-5)

Escherichia coli  ATCC l5389

(ll-6)

Escherichia coli  ATCC l0798

(12-1)

Staphylococcus aureus  IAM I0II/ATCC 6538P/KFCC 35493/KCTC I069

(12-2)

Staphylococcus aureus  IFO 3060/KFCC 32395

(12-3)

Staphylococcus epidermidis  IFO 3762/ATCC I55

(13-1)

Serratia marcescens  IAM II05/ATCC 2I639

(13-2)

Serratia marcescens  IFO 3046

(14-1)

Bacterium cadaveris  ATCC 976

(15-1)

Proteus vulgaris  IAM I025

(15-2)

Proteus vulgaris  IFO 3I67

(15-3)

Proteus vulgaris  IFO 3045

(16-1)

Cellulomonas flavigena  IFO 3753/ATCC 487/KCTC I440

(16-2)

Cellulomonas flavigena  IFO 3747/ATCC 49I/KCTC I44I

(16-3)

Cellulomonas flavigena  IFO I2680/ATCC 486/KCTC I370

(16-4)

Cellulomonas flavigena  ATCC 8I83

(17-1)

Enterobacter aerogenes  IFO I20I0/ATCC I5038

(17-2)

Enterobacter aerogenes  ATCC I3048

(17-3)

Enterobacter cloacae   ATCC 7256

(17-4)

Enterobacter cloacae  ATCC I3047

(18-1)

Mycoplana bullata  IFO I3290/ATCC 4278

(18-2)

Mycoplana dimorpha  ATCC 4279

(19-1)

Vibrio anguillarum  IFO I3266/ATCC I9264

(19-2)

Vibrio metchnikovii  ATCC 7708

(20-1)

Erwinia carotovora  IFO I2380

(20-2)

Erwinia carotovora  IFO 3830/ATCC I5390/KFCC II3I9

(20-3)

Erwinia herbicola  ATCC I4536

(21-1)

Klebsiella pneumonia  ATCC 8308

(21-2)

Klebsiella pneumonia  ATCC 962I/KFCC II788

(22-1)

Aeromonas hydrophila  subsp. anaerogenes

IFO I3282/ATCC I5467

(22-2)

Aeromonas punctata  ATCC III63

(22-3)

Aeromonas salmonicida   ATCC 14174

(23-1)

Alteromonas putrefaciens   ATCC 8071

(23-2)

Alteromonas putrefaciens   ATCC 8072

(23-3)

Alteromonas putrefaciens   ATCC 8073

(24-1)

Kurthia zopfii   IFO 12083/ATCC 6900

(24-2)

Kurthia zopfii   IFO 12084/ATCC 10538

(25-1)

Protaminobacter alboflavus   IFO 3707/ATCC 8458/KCTC 1303

(26-1)

Salmonella schottomuelleri   ATCC 8759

(26-2)

Salmonella enteritidis   IFO 3313

(27-1)

Citrobacter freundii   ATCC 8090

(28-1)

Sporosarcina ureae   ATCC 6473

(29-1)

Alcaligenes metalcaligenes   ATCC 13270

(30-1)

Actinoplanes missouriensis   IFO 13243

(31-1)

Micromonospora purpurea   IFO 13150/ATCC 15853/KFCC 34713

(32-1)

Nocardia erythropolis   IFO 12682

(33-1)

Streptoverticillium roseoverticillatum   IFO 12817

(34-1)

Streptosporangium roseum   IFO 3776

(35-1)

Thermoactinomyces vulgaris   IFO 13605

(36-1)

Streptomyces olivaceus   IFO 12805

(36-2)

Streptomyces fradiae   IFO 12773/ATCC 10745

(36-3)

Streptomyces phaeochromogenes   IFO 12898/ATCC 23945/KCTC 1074

(36-4)

Streptomyces olivochromogenes   IFO 13067

(36-1)

Saccharomyces cerevisiae   ATCC 2601

(37-1)

Mycotorula japonica   OUT 6226

(38-1)

Candida tropicalia   ATCC 14056

The above listed microorganisms should be suitably selected in accordance with the desired ribonucleosides in the present invention.

Among the above listed microorganisms, those of the genus Brevibacterium are typical of the enzyme source of the present invention. Especially the advantages of this invention are shown specifically and notably when a Brevibacterium microorganism, more particularly a Brevibacterium acetylicum microorganism is used as the enzyme source.

Mutant strains derived from the above enumerated strains by mutation can also be used as the enzyme source of the present invention as long as the strains have the desired enzyme activity. Among organisms obtained from these strains by genetic engineering technology, those which have the desired enzyme activity can further be used as the enzyme source of the present invention.

In the case where an enzyme containing substance derived from microorganisms is used as the enzyme source of the present invention, the microorganisms are cultivated by an ordinary method to obtain microbial cells having the desired enzyme activity.

As culture media, those containing an appropriate quantity of carbon source and nitrogen source assimilable by the microorganism and, if necessary, further comprising a metal salt, trace amounts of a growth promoting substance, an antifoaming agent and the like are used.

Specific examples of the culture medium ingredients are saccharides (e.g., glucose and saccharose), natural carbohydrates (e.g., molasses, blackstrap molasses, starch, wheat, bran and rice), alcohols, fatty acids and hydrocarbons; examples of the nitrogen source are meat extract, yeast extract, soybean hydrolyzate, Casamino acid, various amino acids and urea; examples of the inorganic salt are phosphates, hydrochlorides, sulfates and like salts of metals such as zinc, iron, magnesium, sodium, calcium and potassium; and examples of trace amounts of the growth promoting substance are vitamin $B_1$, vitamin $B_2$, pantothenic acid and biotin.

Ordinarily, cultivation is carried out by the liquid culture method such as shake culture, cultivation under aeration and stirring, static culture and continuous culture.

Cultural conditions vary with the species of the particular microorganism and culture medium employed and thus cannot be specified. Ordinarily, the pH is adjusted to the vicinity of neutrality at the initiation of the culture and the microorganism is subjected to culture under the temperature conditions of the order of 20 to 40°C until a sufficient level of the desired enzyme activity can be obtained.

For the enzyme source used in the present invention, organisms having the desired enzyme activity per se or treated organisms obtained by suitably treating such organisms.

The organisms per se are intended herein to mean the organisms with the desired enzyme activity separated from a culture of the organisms by a conventional separation means such as centrifugation, sedimentation, coagulation and washing and not subjected to any of the following treatments. In the case where the organisms employed are microorganisms, the organisms per se are ordinarily cells harvested from the culture.

The treated organisms, on the other hand, are obtained by subjecting the above organisms to ordinary treatments such as mechanical destruction, for example, by Waring blender, French press, homogenizer or mortar; freezing and thawing; drying, for example, by lyophilization, air-drying and acetone-drying; autolysis by treatment with toluene, ethyl acetate and like solvents; enzyme treatment using cell wall lytic enzymes such as lysozyme; ultrasonication; osmotic shock procedure; and chemical treatment with salt solutions, acidic solutions, alkaline solutions, surfactants, chelating agents, etc.; thereby to destroy the organisms, modify the cell walls and/or cell membranes thereof, or extract the fraction having the enzyme activity and, if necessary, subjecting the extract with the enzyme activity to a conventional enzyme purification process - (such as salting out, isoelectric precipitation, organic solvent precipitation, various chromatography treatments, and dialysis) thereby to separate a fraction having the desired enzyme activity.

The enzyme source thus obtained has high nucleoside phosphorylase activity but at the same time has nucleosidase activity which is obstructive to the object of this invention. A primary object of the present invention is to inhibit nucleosidase activity during enzyme reaction by inclusively immobilizing the enzyme source by a specific method.

The specific immobilization method herein involves irradiating a mixture of the enzyme source and a photo-curable resin having a photosensitive radical at any position of the chain structure thereof with actinic rays to cause three-dimensional crosslinking reaction within the photo-curable resin by the photoreaction thereby to immobilize the enzyme source in the gel structure inclusively.

In the case where microbial cells or treated microbial cells are used as the enzyme source, the cells are subjected to immobilization in the form of a uniformly dispersed aqueous suspension which can contain an organic solvent or a suspending agent.

For the photo-curable resin having a photosensitive radical to be used in the immobilization according to the present invention, a resin comprising as its main chain a prepolymer and as a photosensitive radical an ethylenically unsaturated radical such as acryloyl, methacryloyl or cinnamoyl radical. The photosensitive radical can be present not limitedly at the terminal of the prepolymer chain but at any position thereof.

Specific examples of the photo-curable resin are unsaturated urethanes comprising hydroxyalkyl esters of acids having the ethylenically unsaturated radicals such as acrylic acid and methacrylic acid bonded by urethane bond to the hydroxyl groups of the polyalkylene glycols through diisocyanates. The diisocyanates herein include isophorone diisocyanate, xylylene diisocyanate, tolylene diisocyanate and hexamethylene diisocyanate, and the hydroxyalkyl groups include 2-hydroxyethyl group and 2-hydroxypropyl group.

Examples of suitable unsaturated urethanes are photo-curable resin prepolymers comprising as the main chain a prepolymer consisting of polyethylene glycol or polypropylene glycol or a mixture of polyethylene glycol and polypropylene glycol in any proportion and having acryloyl radicals bonded at both the terminals thereof (hereinafter abbreviated to "ENT", "ENTP" and "ENTG" in the order stated.) ENT, ENTP and ENTG are synthesized respectively from 2-hydroxyethyl acrylate, isophorone diisocyanate and polyethylene glycol; 2-hydroxyethyl acrylate, isophorone diisocyanate and polypropylene glycol; and 2-hydroxyethyl acrylate, isophorone diisocyanate and a mixture of polyethylene glycol and polypropylene glycol. (For the structures of ENT and ENTP, see "KOSO KOGAKU (Enzyme Engineering)", Kabushiki Kaisha Tokyo Kagaku Dojin, September 18, 1981; Eur. J. Appl. Microbiol. Biotechnol., 6, 207 (1979); ibid. 6, 325 (1979); and ibid. 8, 143 (1979).)

Further examples of the photo-curable unsaturated resin include a resin having a photosensitive radical introduced into the hydroxyl group on the side chain or the terminals of the main chain of the bone thereof. Reaction methods suitable for the introduction are, for example, esterification, urethanation, acetalization and etherification. The photosensitive radicals to be introduced are typically radicals having ethylenically unsaturated bonds such as acryloyl, methacryloyl, allyl, vinyl ether, vinyl thioether and vinyl amino radicals. These photosensitive radicals may be introduced singly or in a combination of two or more members.

The resin having a hydroxyl group should desirably have a high strength even after photocuring so that the elution or release of the immobilized enzyme or microbial cells can be prevented as much as possible and, for this purpose, high molecular, water-soluble or water-dispersible resins that can per se be utilized as fibers or plastics are preferred. Examples of such resins are polyvinyl alcohols, ethyl cellulose and hydroxypropyl cellulose. A suitable molecular weight range is 20,000 to 500,000 on the number average, preferably 30,000 to 200,000 in view of the viscosity suitable for handling and the mechanical strength.

A method of introducing unsaturated radicals into the polyvinyl alcohols or water-soluble celluloses that can be employed especially advantageously involves introducing N-methylol acrylamide by virtue of the acetalization of the hydroxyl radical. For example, an unsaturated polyvinyl alcohol (PVA) is synthesized by reacting a PVA having a molecular weight of 50,000 to 100,000 on the number average and a saponification value of 85 to 90% with 0.3 to 5 moles per kg of the PVA of N-methylol acrylamide (N-MAM) in an aqueous solution in the presence of an acidic catalyst such as phosphoric acid, sulfuric acid and p-toluenesulfonic acid thereby to cause reaction between the methylol radical of the N-MAM and the hydroxyl radical of the PVA.

The photo-curable resins suitable for use in this invention are not limited to the unsaturated polyurethanes, unsaturated polyvinyl alcohols and unsaturated celluloses enumerated hereinbefore, but unsaturated polyesters, unsaturated acrylic resins, unsaturated polyamides and unsaturated epoxides with photosensitive radicals introduced therein which are similar in function to the unsaturated polyurethanes and the like mentioned above can also be used in this invention with similar results. (See, for example, Japanese Patent Pub. No. 3196/1984.)

The photo-curable resin may be selected by routine experimentation depending upon the species and properties of the particular enzyme source used, and the desired form and properties of the immobilized enzyme.

The method of immobilizing the enzyme source with the above described photo-curable resin inclusively will now be set forth in detail.

The immobilized nucleoside phosphorylase preparation of the present invention is obtained basically by mixing the enzyme source with one or more members of the aforementioned photo-curable resins in mixture and irradiating the mixture with actinic rays to cause crosslinking reaction by the photoreaction.

In the crosslinking reaction, it is preferred to add a photosensitizer or a photo-initiator to the photo-curable resin and then to blend the enzyme source into the resulting mixture.

Any known photosensitizers or photo-initiators can be used and no specific ones are required. For example, $\alpha$-carbonyl alcohols such as benzoin and acetoin, acyloin ethers such as benzoin methyl ether, benzoin ethyl ether, benzoin propyl ether, anisoin ethyl ether and pivaloin ethyl ether, $\alpha$-substituted acyloins such as $\alpha$-methylbenzoin and $\alpha$-methoxybenzoin, polycyclic aromatic compounds such as naphthol and hydroxyanthracene, azoamide compounds such as 2-cyano-2-butylazoformamide, or metal salts such as uranyl nitrate and ferric chloride can be used advantageously.

In the crosslinking reaction, one or more members of a saccharide (e.g., alginate) solution, water, buffer and an organic solvent may be added to the mixture mentioned previously for dilution purposes.

After or while the mixture thus obtained is formed into any desired shape, the mixture is irradiated with actinic rays to cause crosslinking reaction thereby to obtain an immobilized enzyme source, i.e. immobilized preparation. After immobilization, the immobilized enzyme source may further be formed into any desired shape by cutting, pulverization or some other suitable method.

Prior to or during irradiation with actinic rays, forming or shaping can be carried out by any suitable method. For example, the mixture is blended with a saccharide such as alginate or carrageenan according to the desired purpose, and then applied, spread or laminated on the surface of a support of any desired shape, impregnated into a support penetrable to the mixture (e.g., fibrous support) or coagulated with a coagulant such as calcium chloride or potassium chloride in the case where a saccharide is used in combination, or the aforesaid blend of the mixture with a saccharide is allowed to flow down freely in the coagulated solution.

The support herein may be of any material suitable for the purpose set forth above. For example, synthetic resins, synthetic fibers, natural fibers, ceramics, and metals can be used.

The immobilized preparation to be subjected to the enzymatic reaction can be formed into any shape according to any of the forming methods described hereinabove, for example, into the form of films including sheets, layers, granules, cylinders, linear materials, fibers and rings.

The light source of actinic rays for use in the crosslinking reaction may be any instrument that emits light rays in the range of 220 to 700 nm, preferably 250 to 600 nm in wavelength. Examples of such light sources are low-pressure mercury lamps such as fluorescent chemical lamps and black light fluorescent lamps, high-pressure mercury lamps, fluorescent lamps, xenon lamps, carbon arc lamps and sunlight. Irradiation with actinic rays required for the crosslinking reaction is ordinarily carried out from I to I0 minutes. The irradiation can be performed in an aqueous solution, an atmosphere of inert gas and/or under low temperature conditions.

The immobilized preparation thus obtained is contacted with a substrate solution which will be described hereinafter in a reactor suitable for the shape of the preparation, for example, in a reactor into which the preparation can be charged or in which the preparation can form a film, be fluidized, suspended or stirred, to cause enzymatic reaction.

The enzymatic reaction of the present invention, i.e., the reaction between the base donor and the ribose compound in the presence of the nucleoside phosphorylase preparation, is known in the art - (Japanese Patent Laid-Open Pub. No. I90396/I983, Japanese Patent Laid-Open Pub. No. 2I6696/I983 and Japanese Patent Laid-Open Pub. No. I43599/I984).

The substrates in the enzymatic reaction of the present invention are the base donor and the ribose compound.

The base donor may be selected according to the desired species of ribonucleosides, for example, an aromatic heterocyclic base or its derivatives capable of forming N-glycosidic linkage with the ribose moiety of the ribose compound by the action of nucleoside phosphorylase. Examples of the aromatic heterocyclic base include a purine derivative, pyrimidine derivative, triazole derivative, imidazole derivative, deazapurine derivative, azapurine derivative, azapyrimidine derivative and pyridine derivative. The base donor, in additon to the aromatic heterocyclic base per se, may also be the derivatives thereof that are capable of transferring the aromatic heterocyclic base to the ribose moiety of the ribose compound such as nucleosides or nucleotides having sugar residues except for ribofuranosyl residue.

Specific examples of the aromatic heterocyclic base are purine derivatives having a substituent or substituents (e.g., amino group, substituted amino group, hydroxyl group, oxo group, mercapto group, acyl group, alkyl group, substituted alkyl group, alkoxyl group and halogen atom) at one or more of the I-, 2-, 6- and 8-positions of purine, such as adenine, guanine, hypoxanthine, xanthine, 6-mercaptopurine, 6-thioguanine, $N^6$-alkyl-or acyladenine, 2-alkoxyadenine, 2-thioadenine and 2,6-diaminopurine; pyrimidine derivatives having the same substituents as are enumerated above at one or more of the 2-, 4-and 5-positions of pyrimidine, such as cytosine, uracil, thymine, 5-halogenouracils (e.g., 5-fluorouracil, and 5-iodouracil), 5-halogenocytosines (e.g., 5-fluorocytosine), 5-trihalogenomethyluracils (e.g., 5-trifluoromethyluracil), 4-thiocytosine, 4-thiouracil, $N^4$-acylcytosine and 5-halogenovinyluracils; I,2,4-triazole derivatives having a substituent at the 3-position of I,2,4-triazole, such as I,2,4-triazole-3-carboxamide, I,2,4-triazole-3-carboxylic acid and alkyl esters of I,2,4-triazole-3-carboxylic acid; imidazole derivatives having substituents at the 4-and 5-positions of imidazole, such as 5-amino-4-imidazole carboxamide and 4-carbamoyl-imidazolium-5-oleate; deaza compound derivatives, such as I-deazaadenine, 3-deazaadenine, 3-

11

.deazaguanine, 7-deazaadenine and 7-deazaguanine or those compounds that have the same substituents as have been enumerated for the purine derivatives; azapurine derivatives such as 8-azaadenine; azapyrimidine derivatives such as 5-azathymine, 5-azacytosine and 6-azauracil; and pyridine derivatives such as 3-deazauracil, nicotinic acid and nicotinamide.

The ribose compound, on the other hand, may be a ribose derivative capable of forming N-glycosidic linkage between the ribose moiety thereof and the base moiety of the base donor by the action of nucleoside phosphorylase. Ordinarily, ribonucleosides, ribonucleotides or ribose-l-phosphate are used.

Specific examples of the ribonucleosides are adenosine, guanosine, inosine, xanthosine, uridine and cytidine, while specific examples of the ribonucleotides are monophosphate esters, diphosphate esters or triphosphate esters of the ribonucleosides at the 2'-, 3'-or 5'-position thereof.

By contacting a reaction solution containing the base donor and the ribose compound with the immobilized nucleoside phosphorylase preparation, ribonucleosides corresponding to the base donor used can be synthesized enzymatically.

A reaction solution comprising the base donor and one or more of the ribose compounds dissolved or suspended in water or a buffer is suitable and the solution may further comprise a phosphate donor or an organic solvent as necessary. Since the enzymatic reaction of the present invention relies basically on the action of nucleoside phosphorylase, the reaction solution must contain phosphate ion. In the case where a nucleotide is used as a substrate, however, phosphate ion is produced by the action of phosphatase or nucleotidase contained in the enzyme source, or a phosphate ion donor may sometimes be contained in the enzyme source per se, so that a phosphate ion donor need not necessarily be added from outside the reaction system.

For the phosphate ion donors, any of those capable of dissociating into phosphate ion in the reaction solution can be used. For example, free phosphoric acid per se or phosphate salts (e.g., salts of alkali metals such as sodium and potassium, salts of alkaline earth metals such as calcium and magnesium, and ammonium salt) are used advantageously. Any system that can release phosphate ion in the reaction solution, for example, a combination of any phosphate ester derivative and phosphatase or a combination of a nucleotide and nucleotidase, can also be utilized as the phosphate ion donor.

Ordinarily, the reaction of the present invention proceeds efficiently in the temperature range of from 35 to 70°C, the reaction temperature of from 40 to 65°C being especially preferred. At 35°C or lower temperatures, the reaction rate is low, while various intervening enzymes are highly active, resulting in poor reaction efficiency. Conversely, reaction temperatures of 70°C or higher are liable to lower also the nucleoside phosphorylase activity.

The reaction solution may be maintained at a pH of ordinarily from 4 to l0, preferably from 6 to 8. In the event that the pH fluctuates during reaction, an acid or an alkali may be added to control the pH to fall within a preferred range.

After completion of the reaction, the immobilized nucleoside phosphorylase preparation is separated from the reaction solution to isolate and purify the desired ribonucleoside.

The ribonucleoside thus produced can be isolated and purified by a known method or a modified known method. For example, various types of chromatography such as ion exchange chromatography, adsorption chromatography, partition chromatography and gel filtration, methods utilizing the distribution between two liquid phases such as countercurrent distribution and countercurrent extraction, methods utilizing the difference in solubility such as concentration, cooling and addition of organic solvents and like conventional isolation and purification methods may be employed singly or in combination as necessary.

## Examples

In order to indicate more fully the nature and utility of this invention, the following specific examples of practice constituting preferred embodiments of the invention and reference examples are set forth, it being understood that these examples are presented as illustrative only and not intended to limit the scope of the invention.

In the examples, the end product ribonucleosides were analyzed by high-speed liquid chromatography.

Example 1

A culture medium containing 1.5% of yeast extract was adjusted to a pH of 7.0 and sterilized at 120°C for 15 minutes. Brevibacterium acetylicum AT-6-7 (FERM P-6305) was inoculated into the medium thus sterilized and subjected to shake culture at 28°C for 24 hours. Microbial cells were recovered from the culture broth, washed with 0.1 M tris buffer (pH 7.0) and then centrifuged to obtain washed cells.

2.0 g of the washed cells thus obtained were suspended in 1.0 ml of 0.1 M tris buffer (pH 7.0), and the suspension was added to a separately prepared resin solution of 8.0 g of a photo-curable resin (ENT-2000, available from Kansai Paint K.K., Japan) with 0.08 g of benzoin ethyl ether as a photo-initiator. The suspension and the resin solution were thoroughly mixed and thereafter spread over a transparent film which was then irradiated with actinic rays of around 360 nm for 3 minutes simultaneously on the front and back surfaces thereof to obtain a photopolymer.

A portion containing 0.2 g of cells was excised from the immobilized cell film, comminuted, placed into 10 ml of an aqueous solution (substrate solution) containing 40 mM 1,2,4-triazole-3-carboxamide, 60 mM disodium 5'-uridylate and 8 mM monopotassium phosphate, and stirred at 45°C for 72 hours to produce ribavirin (1-$\beta$-D-ribofuranosyl-1,2,4-triazole-3-carboxamide).

As a control, 0.2 g of the aforementioned washed cells per se were added to equal volume of the above substrate solution to cause reaction under the same conditions. The overall results are shown in Table 1.

In the reaction described above, the mole of the product ribavirin and that of the by-product uracil should be equal from the stoichiometric point of view. In fact, however, the strain used has nucleosidase activity together with nucleoside phosphorylase activity so that all the substrate 5'-uridylic acid is not utilized for the production of ribavirin but part thereof is decomposed to produce uracil, resulting in the mole ratio of higher than 1.0 of the uracil to the ribavirin produced in the reaction solution.

In the following Tables, the conversion (%) refers to the mole percent of ribavirin produced to 1,2,4-triazole-3-carboxamide used as a substrate, and the mole ratio refers to the mole ratio (uracil/ribavirin mole ratio) of a by-product uracil to ribavirin produced.

## Table 1

| Reaction Time (hr) | Immobilized Cell (Present Invention) | | Live Cell (Control) | |
|---|---|---|---|---|
| | Conversion (%) | Mole Ratio | Conversion (%) | Mole Ratio |
| 24 | 80.4 | 1.05 | 77.0 | 1.28 |
| 48 | 88.9 | 1.08 | 72.3 | 1.48 |
| 72 | 90.0 | 1.10 | 71.4 | 1.97 |

As is apparent from the above data indicating that the "mole ratio" is reduced in accordance with the method of the present invention as compared with the control, the nucleosidase activity is inhibited. Further, ribavirin once produced in the control case was decomposed as the reaction proceeded while ribavirin produced by the present method was not decomposed but remained stable.

Example 2

Immobilized cells prepared as in Example 1 were charged into a 115-ml (inner volume) column provided with a jacket, and a substrate solution of the same composition as that of the solution used in Example 1 was passed therethrough at a rate of 40 ml/hr. to cause ribavirin producing reaction. The results obtained are presented in Table 2 in terms of the conversion (%) into ribavirin and uracil/ribavirin mole ratio.

## Table 2

| Solution Passing Time (hr.) | Immobilized Cell Column | |
| --- | --- | --- |
| | Conversion (%) | Mole Ratio |
| 24 | 92.30 | 1.13 |
| 120 | 93.67 | 1.11 |
| 240 | 92.93 | 1.13 |
| 360 | 88.92 | 1.13 |

As noted from Table 2, the conversion was not reduced materially, nor did the "mole ratio" fluctuate in the case of the immobilized cell column.

### Example 3

Ribavirin producing reactions were carried out by the repeated batch reaction method respectively using immobilized cells prepared by the procedure of Example I from 0.5 g of washed cells obtained as in Example I and 0.5 g of live cells to compare the stability of the conversions.

The reactions were carried out at 45°C with the same volumes of the same substrate solutions as were used in Example I, and the reaction solutions were analyzed, the enzyme sources collected by filtration, and the substrate solutions fed every 23 hours. The results obtained are summarized in Table 3.

## Table 3

| Time | Conversion (%) | |
| --- | --- | --- |
| | Immobilized Cell | Live Cell |
| 1 | 90.33 | 90.00 |
| 4 | 82.92 | 81.95 |
| 6 | 82.82 | 64.08 |
| 8 | 80.00 | 23.26 |
| 10 | 82.14 | - |
| 12 | 81.11 | - |

The above data show that the conversion for the live cells was reduced drastically at the sixth reaction cycle et seq. while the conversion for the immobilized cells was stable even after the reaction was repeated twelve times.

### Example 4

Immobilized cells (0.l6 g of live cells having been immobilized) prepared by the procedure of Example I were placed into l0 ml of a substrate solution (pH 7.0) containing 20 mM hypoxanthine, 30 mM uridine and 30 mM monopotassium phosphate which was then stirred at 50°C for l8 hours to cause inosine producing reaction. The conversion of hypoxanthine into inosine was 66.3%.

14

As a control, a reaction was carried out under the above described conditions except that the same quantity of live cells were employed, resulting in a conversion into inosine of 58.5%.

Example 5

The procedure of Example 3 was repeated except that the photo-curable resin ENT-2000 was replaced by ENT-2000N * (available from Kansai Paint K.K., Japan) to cause ribavirin producing reaction. The results obtained are shown in Table 4.

* A photo-curable resin wherein the main chain comprises polyethylene glycol and the ethylenically unsaturated radical is methacryloyl radical.

Table 4

| Time | Conversion (%) |
|---|---|
| 1 | 92.18 |
| 2 | 92.92 |
| 5 | 86.87 |
| 7 | 84.28 |
| 9 | 82.67 |

As is apparent from the above data, the reaction was found to proceed stably also with ENT-2000N.

Example 6

5.0 g of washed cells obtained as in Example I were suspended in I.0 ml of 0.IM tris buffer (ph 7.0), and to this suspension was added 2.0 ml of 3% sodium alginate solution to obtain a homogeneous suspension.

A resin solution of I0.0 g of a photo-curable resin (ENTG-3800 * , available from Kansai Paint K.K., Japan) with 0.I g of benzoin ethyl ether as a photo-initiator was added to the cell suspension and thoroughly mixed therewith. The mixture thus obtained was taken into a syringe having a tube of an inner diameter of I.45 mm and dropped into 0.I M calcium chloride solution (pH 7.0) to prepare particulates.

These particulates were placed on a laboratory dish and irradiated with actinic rays of the order of 360 nm for 5 minutes simultaneously from above and below to obtain immobilized particulate cells which were photopolymers.

The immobilized particulate cells containing 0.5 g of cells were taken up and subjected to repeated batch reaction as in Example 3. The results are set forth in Table 5 below.

*8:2 mixture of polyethylene glycol and polypropylene glycol

15

Table 5

| Time | Conversion (%) |
|------|----------------|
| 1 | 96.22 |
| 2 | 91.39 |
| 4 | 87.59 |
| 6 | 85.14 |
| 8 | 83.26 |

The above data demonstrate that the reaction proceeded stably again with ENTG-3800 as well as with the immobilized particulate cells.

Example 7

Erwinia carotovora (IFO 12380) was inoculated into a liquid medium (pH 7.0) containing 1.5% of a yeast extract, and subjected to shake culture at 28°C for 24 hours.

Immobilized cells of this strain were prepared by the procedure of Example I using ENTG-3800. With these immobilized cells, the uridine producing reaction by the use of a substrate solution (pH 7.0) containing 20 mM uracil, 30 mM inosine and 30 mM monopotassium phosphate was carried out.

Table 6

| Immobilized Cell (Present Invention) | | Live Cell (Control) | |
|---|---|---|---|
| Conversion (%) | Hypoxanthine/ Uridine Mole Ratio | Conversion (%) | Hypoxanthine/ Uridine Mole Ratio |
| 18.0 | 2.0 | 15.1 | 4.6 |

Example 8

Kurthia zopfii (IFO 12083) was inoculated into a liquid medium (pH 7.0) containing 1.5% of a yeast extract, and subjected to shake culture at 28°C for 24 hours.

16

Immobilized cells of this strain were prepared by the procedure of Example I using ENTG-3800. With these immobilized cells, the ribavirin producing reaction described in Example 3 by the use of a substrate solution (pH 7.0) containing 32 mM triazole, 48 mM disodium 5'-uridylate and 6.4 mM monopotassium phosphate was carried out.

### Table 7

| Immobilized Cell (Present Invention) | | Live Cell (Control) | |
|---|---|---|---|
| Conversion (%) | Uracil/Ribavirin Mole Ratio | Conversion (%) | Uracil/Ribavirin Mole Ratio |
| 28.2 | 1.1 | 24.7 | 1.4 |

### Example 9

Brevibacterium acetylicum (ATCC 953) was cultivated as in Example 7 to obtain microbial cells. Immobilization of the cells was carried out by the procedure of Example I using ENT-2000 and ENTG-3800, respectively. With these immobilized cells, the ribavirin producing reaction was individually carried out at 45°C for 20 hours by using the substrate solution described in Example I. The results are set forth in Table 8 below.

### Table 8

| Enzyme Preparation | Photo-Curable Resin | Conversion (%) | Mole Ratio |
|---|---|---|---|
| Immobilized Cell | ENT-2000 | 79.6 | 1.23 |
| | ENTG-3800 | 79.5 | 1.42 |
| Live Cell | - | 73.1 | 2.13 |

Reference Example 1

The following experiment was conducted in order to compare the nucleosidase activity between the immobilized cells of the present invention and live cells.

Cultured cells of the same strain as was used in Example I were immobilized by the procedure of Example I to obtain immobilized cells corresponding to 0.5 g of live cells. The immobilized cells thus obtained were placed into 10 ml of 30 mM uridine solution (pH 7.0) which was then stirred at 45°C, and the uracil production rate was measured as the reaction proceeded.

Separately, 0.5 g of live cells of the same strain as was mentioned above were subjected to reaction under the same conditions as were employed in the above reaction, and the uracil production rate was measured as the reaction proceeded.

The results obtained are set forth in Table 9.

## Table 9

| Reaction Time (hr.) | Uracil Production Rate (%) | |
| --- | --- | --- |
| | Immobilized Cell | Live Cell |
| 0.5 | 0 | 5.6 |
| 1.5 | 0 | 6.9 |
| 2.5 | 0 | 7.6 |
| 19.0 | 0 | 11.0 |
| 48.0 | 0 | 14.5 |

As is apparent from the above Table, uridine was not decomposed at all even after 48 hours' reaction when the immobilized cells were used, while uridine was decomposed incrementally with the elapse of time when the live cells were employed.

Reference Example 2

For reference, the following example is shown in which ribonucleosides were produced using immobilized cells prepared in accordance with a method other than the specific immobilization method of the present invention.

The same strain as was used in Example I was inoculated into 1 liter of a 2% bouillon medium and subjected to shake culture at 30°C for 22 hours. The culture broth is subjected to centrifugation to obtain live cells.

To the live cells thus obtained were added 20 ml of solution A (an aqueous solution of 24 ml of IN hydrochloric acid, 3.425 g of Tris and 0.23 ml of N, N, N′, N′-tetramethylenediamine diluted with water to 100 ml), 20 ml of Solution B (100 ml of an aqueous solution containing 30 g of acrylamide and 0.8 g of N, N-methylene bis-acrylamide) and 40 ml of Solution C (200 ml of an aqueous solution containing 0.3 g of ammonium persulfate) and the resultant mixture was left standing to immobilize the cells. The cells thus immobilized were then comminuted in a homogenizer to obtain 180 ml of immobilized cells.

To 10 ml of these immobilized cells was added 20 ml of a substrate solution (pH 7.0) containing 20 mM 1, 2, 4-triazole-3-carboxamide, 20 mM inosine and 25 mM monopotassium phosphate, and the mixture was reacted at 60°C for 24 hours. Upon analysis of the reaction solution, 62.89% of ribavirin was found to be produced.

Separately, live cells were subjected to reaction under the same conditions, resulting in a ribavirin production rate of 65.44%.

## Claims

1. In a process for producing ribonucleosides comprising reacting a base donor with a ribose compound by the action of nucleoside phosphorylase to form N-glycosidic linkage between the base moiety of the base donor and the ribose moiety of the ribose compound thereby to produce ribonucleosides, the nucleoside phosphorylase being a nucleoside phosphorylase preparation having nucleosidase activity in addition to the nucleoside phosphorylase activity, the improvement which comprises the use, as the nucleoside phosphorylase preparation, of an immobilized nucleoside phosphorylase preparation obtained by subjecting a mixture of an enzyme source having nucleosidase activity in addition to nucleoside phosphorylase activity and a photo-curable resin having a photosensitive radical at any position of the prepolymer chain structure thereof to irradiation with actinic rays, whereby the activity of the nucleosidase concomitantly present in the enzyme source is inhibited.

2. The process of Claim I wherein said ribonucleosides are those selected from the group consisting of 9-β-D-ribofuranosyl purines, I-β-D-ribofuranosyl pyrimidines, I-β-D-ribofuranosyl-I,2,4-triazoles, I-β-D-ribofuranosyl imidazoles, deazapurine β-D-ribofuranosides, azapurine β-D-ribofuranosides, azapyrimidine β-D-ribofuranosides, and I-β-D-ribofuranosyl pyridines.

3. The process of Claim 2 wherein said I-β-D-ribofuranosyl-I,2,4-triazoles are those selected from the groups consisting of I-β-D-ribofuranosyl-I,2,4-triazole-3-carboxamide, I-β-D-ribofuranosyl-I,2,4-triazole-3-carboxylic acid and I-β-D-ribofuranosyl-I,2,4-triazole-3-alkyl carboxylate.

4. Claim 3 wherein said I-β-D-ribofuranosyl-I,2,4-triazole is I-β-D-ribofuranosyl-I,2,4-triazole-3-carboxamide (ribavirin).

5. The process of Claim I wherein said enzyme source is a microorganism per se in cell form.

6. The process of Claim 5 wherein said microorganisms is selected from the group consisting of bacteria, actinomycetes and yeast.

7. The process of Claim 6 wherein said bacteria are microorganisms of the genera selected from the group consisting of Brevibacterium, Erwinia and Kurthia.

8. The process of Claim 6 wherein strains of said bacteria are derived from Brevibacterium acetylicum .

9. The process of Claim 8 wherein said bacteria strain is Brevibacterium acetylicum AT-6-7 ATCC 393II/FERM P-6305.

I0. The process of Claim I wherein said photo-curable resin having a photosensitive radical is a resin comprising as its main chain a prepolymer and as a photosensitive radical an ethylenically unsaturated radical.

11. The process of Claim I0 wherein said photosensitive radical is selected from the group consisting of acryloyl, methacryloyl, acryloylamino, allyl, vinyl ether, vinyl thioether and vinyl amino radicals.

12. The process of Claim I0 wherein said resin is an unsaturated urethane comprising a hydroxyalkyl ester of an acid having an ethylenically unsaturated radical bonded by urethane bond to the hydroxyl group of polyalkylene glycol through a diisocyanate.

13. The process of Claim 12 wherein said diisocyanate is selected from the group consisting of isophorone diisocyanate, xylylene diisocyanate, tolylene diisocyanate and hexamethylene diisocyanate.

14. The process of Claim 12 wherein said unsaturated urethane is a photo-curable resin prepolymer comprising as the main chain a prepolymer consisting of polyethylene glycol or polypropylene gylcol or a mixture of polyethylene glycol and polypropylene glycol in any proportion and having acryloyl radicals bonded at both the terminals thereof.

15. The process for Claim 10 wherein said main chain is selected from the group consisting of polyvinyl alcohols, ethyl cellulose and hydroxypropyl cellulose.

16. The process of Claim I wherein the reaction between a base donor and a ribose compound by the action of nucleoside phosphorylase is carried out in the temperature range of from 35 to 70°C.

17. The process of Claim 16, wherein said reaction is carried out in a solution maintained at a pH of from 4 to I0.

18. An immobilized nucleoside phosphorylase preparation produced by a process which comprises subjecting a mixture of a Brevibacterium acetylicum microorganism and a photo-curable resin having a photosensitive radical at any position of the prepolymer chain structure thereof to irradiation with actinic rays.